(19) Europäisches Patentamt
European Patent Office
Office européen des brevets

(11) EP 1 277 842 B1

(12) **EUROPEAN PATENT SPECIFICATION**

(45) Date of publication and mention of the grant of the patent:
**29.10.2008 Bulletin 2008/44**

(51) Int Cl.:
***C12Q 1/68*** *(2006.01)* ***G01N 33/50*** *(2006.01)*

(21) Application number: **02015665.9**

(22) Date of filing: **17.07.2002**

(54) **Method for quantifying nucleic acid by cell counting**

Verfahren zur Quantifizierung von Nukleinsäuren mittels Bestimmung der Zellzahl

Méthode pour la quantification d'acides nucléiques basée sur la numération cellulaire

(84) Designated Contracting States:
**AT BE BG CH CY CZ DE DK EE ES FI FR GB GR IE IT LI LU MC NL PT SE SK TR**

(30) Priority: **17.07.2001 JP 2001216568**

(43) Date of publication of application:
**22.01.2003 Bulletin 2003/04**

(73) Proprietor: **FUJIFILM Corporation**
**Minato-ku**
**Tokyo (JP)**

(72) Inventors:
- **Sudo, Yukio,**
  **Fuji Photo Film Co., Ltd.**
  **Asaka-shi,**
  **Saitama 351-8585 (JP)**
- **Some, Masato,**
  **Fuji Photo Film Co., Ltd.**
  **Ashigarakami-gun,**
  **Kanagawa 258-8538 (JP)**

(74) Representative: **Hiebl, Inge Elisabeth et al**
**Kraus & Weisert**
**Patent- und Rechtsanwälte**
**Thomas-Wimmer-Ring 15**
**80539 München (DE)**

(56) References cited:
- **DE BLESER PIETER J ET AL: "Transforming growth factor-beta gene expression in normal and fibrotic rat liver" JOURNAL OF HEPATOLOGY, vol. 26, no. 4, 1997, pages 886-893, XP008061536 ISSN: 0168-8278**
- **MURATA SHIN'ICHI: "Correlated analysis of nuclear DNA content and nuclear morphological features in the thyroid hyperplasias and tumors using image cytometry." KYOTO FURITSU IKA DAIGAKU ZASSHI (JOURNAL OF KYOTO PREFECTURAL UNIVERSITY OF MEDICINE), VOL. 100, NO. 3, PP. 311-331. JOURNAL CODE: Z0618A (FIG. 12, TBL. 3, REF. 47) ISSN: 0023-6012, 1991, XP008061479**
- **SOOST H-J ET AL: "COMPARISON OF PAPANICOLAOU STAINING AND FEULGEN STAINING FOR AUTOMATED PRE SCREENING" ANALYTICAL AND QUANTITATIVE CYTOLOGY, vol. 5, no. 1, 1983, pages 61-66, XP008061611 ISSN: 0190-0471**
- **FINK L ET AL: "Immunostaining for cell picking and real-time mRNA quantitation" AMERICAN JOURNAL OF PATHOLOGY, PHILADELPHIA, PA, US, vol. 157, no. 5, November 2000 (2000-11), pages 1459-1466, XP002327655 ISSN: 0002-9440**
- **VENET D ET AL: "Separation of samples into their constituents using gene expression data" BIOINFORMATICS, OXFORD UNIVERSITY PRESS, OXFORD,, GB, vol. 17 Suppl 1, June 2001 (2001-06), pages S279-S287, XP002327802 ISSN: 1367-4803**
- **ROSENBERG S A ET AL: "AUTOMATIC IDENTIFICATION AND MEASUREMENT OF CELLS BY INST COMPUTER HUMAN NEOPL MALIGNANT CERVICAL CELLS" SCIENCE (WASHINGTON D C), vol. 163, no. 3871, 1969, pages 1065-1066, XP008061691 ISSN: 0036-8075**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 277 842 B1

SEQUENCE LISTING

<110> Fuji Photo Film Co.Ltd.,

<120> A method for measuring an amount of expression of genes

<130> FA2147A

<160> 31

<210> 1
<211> 23
<212> DNA
<213> Artificial DNA

<400> 1
aaaggagttc cggggcataa aag 23

<210> 2
<211> 18
<212> DNA
<213> Artificial DNA

<400> 2
tggcagcatt ccttgtgg 18

<210> 3
<211> 18
<212> DNA
<213> Artificial DNA

<400> 3
tctcagcagc agcacgac 18

<210> 4
<211> 24
<212> DNA
<213> Artificial DNA

<400> 4
aacatttgct gcccactttt ccta 24

<210> 5
<211> 17
<212> DNA
<213> Artificial DNA

<400> 5
ctcggcaaag caggtct 17

<210> 6
<211> 20
<212> DNA
<213> Artificial DNA

<400> 6
ttaattagcc cacagaaact 20

<210> 7
<211> 24
<212> DNA
<213> Artificial DNA

<400> 7
gcaaagagtg ggtagggaca ggag 24

<210> 8
<211> 22
<212> DNA
<213> Artificial DNA

<400> 8
cagggggcag tcttggcaca cc 22

<210> 9
<211> 24
<212> DNA
<213> Artificial DNA

<400> 9
gaaggtgtgg cgcaggtcgt agtg 24

<210> 10
<211> 26

<212> DNA
<213> Artificial DNA

<400> 10
tcaggcactt tcattaacag gcacat 26

<210> 11
<211> 26
<212> DNA
<213> Artificial DNA

<400> 11
cctccatcgg acatgtcaca ataaac 26

<210> 12
<211> 27
<212> DNA
<213> Artificial DNA

<400> 12
aagtaacaaa ggcaagtgag aagaatg 27

<210> 13
<211> 26
<212> DNA
<213> Artificial DNA

<400> 13
acccatgcca ggtgtaccag acaatc 26

<210> 14
<211> 26
<212> DNA
<213> Artificial DNA

<400> 14
ttggggaata ttcaactcgt agaaat 26

<210> 15
<211> 26
<212> DNA
<213> Artificial DNA

<400> 15
gtgctgggtg aaccttctga tgctaa 26

<210> 16
<211> 23
<212> DNA
<213> Artificial DNA

<400> 16
ccagcctggc cgatagaatg aga 23

<210> 17
<211> 25
<212> DNA
<213> Artificial DNA

<400> 17
acttgagccc aggagtttga gacca 25

<210> 18
<211> 21
<212> DNA
<213> Artificial DNA

<400> 18
gggggccaag gcagaaggat t 21

<210> 19
<211> 26
<212> DNA
<213> Artificial DNA

<400> 19
tggatagctg cccattgcaa gttaca 26

<210> 20
<211> 25
<212> DNA
<213> Artificial DNA

<400> 20
gggccacatt ttcttcttgc tccta 25

<210> 21
<211> 20
<212> DNA
<213> Artificial DNA

<400> 21
tccaccccca aagaaaatac 20

<210> 22
<211> 24
<212> DNA
<213> Artificial DNA

<400> 22
tctttcttga tggcaggcac tacc 24

<210> 23
<211> 25
<212> DNA
<213> Artificial DNA

<400> 23
cagttcccat gtggcagcag tagtg 25

<210> 24
<211> 26
<212> DNA
<213> Artificial DNA

<400> 24
cagaggctct gctgatttca cttatg 26

<210> 25
<211> 23
<212> DNA
<213> Artificial DNA

<400> 25
caccaacacc agcagcaaca atc

<210> 26
<211> 23
<212> DNA
<213> Artificial DNA

<400> 26
agggcttgtc agttggacga gtt 23

<210> 27
<211> 23
<212> DNA
<213> Artificial DNA

<400> 27
ggcgatcaat cttttccttc agc 23

<210> 28
<211> 26
<212> DNA
<213> Artificial DNA

<400> 28
tgagaccgtt cttccaccac tgatta 26

<210> 29
<211> 22
<212> DNA
<213> Artificial DNA

<400> 29
cgcggttggc cttggggttc ag 22

<210> 30
<211> 26
<212> DNA
<213> Artificial DNA

<400> 30
gcctagaagc atttgcggtg gacgat 26

<210> 31
<211> 19
<212> DNA
<213> Artificial DNA

<400> 31
ggctgcctcc acccactcc 19

## Description

Technical Field

The present invention relates to a method for analyzing a nucleic acid, and more particularly to a method for correcting analyzed data of the nucleic acid. The present invention also relates to a method for improving accuracy in the process for analyzing DNA or mRNA such as DNA microarrays or DNA chips. More specifically, the present invention relates to a method for measuring a gene in a target cell existing in a specimen (such as tissue slice) comprising two or more types of cells.

Background Technique

It is significant to identify and detect differences in the number of copies of genes or the expression level among given groups of cells for the purpose of research and detection of diseases based on the gene abnormality. For example, many malignant tumors are known to involve the activation of oncogene and/or the inactivation of tumor suppressing genes. Identification of the copy number or the changed expression level of genes associated with these diseases is useful in the early detection of the diseases, the prediction of therapeutic efficiency and the like.

Cytogenetic analysis is one method for detecting an amplified or deleted chromosome region. However, cytogenetic analysis cannot detect rearrangements smaller than 10 Mb (an approximate band width in Giemsa-stained chromosome) and it is difficult to cytogenetically analyze a complicated karyotype having many translocations and other gene alterations.

Construction of a system for simultaneously monitoring the expression level of all the genes in the cell is expected to be useful for elucidation of diseases caused by the gene abnormality, as well as elucidation of general life phenomena.

Intracellular gene expression has been analyzed by, for example, Northern blotting in which RNA extracted from cells is immobilized on a filter and is detected by using a gene-specific probe or RT-PCR which utilizes a primer specific to each gene. Even with these methods, however, it is impossible to simultaneously monitor all the genes, which are deduced to be present in amounts of at least several thousands in microorganisms and about 100,000 in humans. A method of analysis using DNA microarrays has been recently developed as a method for analyzing gene expression in a more simple and direct manner (DeRiesil, JL et al., Science (1997) 278 : 680-686; and Lashkari DA et al., Proc. Natl. Acad. Sci. USA (1997) 94: 13057-13062).

In the DNA microarray technique, several thousands to several tens of thousands DNA spots are prepared on a slide glass, and the target DNA prepared from RNA to be analyzed is hybridized, thereby measuring the transcription level of each gene using the hybridization intensity as an index. At present, instruments for preparing a microarray (spotter or arrayer) or a detector (scanner) are commercially available.

As described above, techniques for analyzing DNA, RNA and the like has been progressively developed. Also, the technique per se for extracting nucleic acids such as DNA, RNA or mRNA has been greatly improved together with the progress of analytical techniques. Even though the steps for detection and extraction have been greatly developed, it is still difficult to obtain accurate data if the purity of cell or tissue specimens is low.

When nucleic acid is extracted from a tissue slice which is a specimen, it is difficult to prevent contamination of lymphocytes by the conventional method. Similarly, in the case where cancer tissue slice is used, it is difficult to prevent contamination of normal cells in the cancer lesion tissue slice. Such contamination of unintended cells and tissues into the targeted specimen tissue significantly lowers the reliability of final measured data (measured data of the nucleic acid). This problem can be solved to some extent by what is called a "microdissection method". However, this method also introduces new problems such as lowered assay sensitivity, and complicated handling of specimen.

De Bleser, P.J., et al, Journal of Hepatology 1997; 26:886-893, describes transforming growth factor-β gene expression in normal and fibrotic rat liver wherein the expression of TGF-$\beta_{1,2,3}$ transcripts was investigated using Northern hybridization analysis. Hybridization signals were quantified by scanning densitometry and corrected for: (i) differences in extractable RNA per cell type, (ii) signal contribution from contaminating cells and (iii) differences in loading, capillary transfer and hybridization.

Disclosure of the Invention

The object of the present invention is to solve the aforementioned problems of the prior art. More specifically, an object of the present invention is to provide a method for correcting data variations caused by tissues and cells other than the targeted tissues in the measurement of tissues and cellular nucleic acids (DNA, mRNA, RNA). Another object of the present invention is to improve the data reliability of a system for analyzing nucleic acids such as DNA microarrays or DNA chips.

The present inventors have earnestly studied to attain the above objects, and have found that the reliability of the quantified data on the nucleic acid can be increased by measuring the cell types contained in the tissue as a specimen and correcting the quantified data of the nucleic acid based on the distribution of the cell types, thereby completing the present invention.

According to the present invention, there is provided a method for quantifying the amount of nucleic acid existing in a specimen which comprises steps of:

(1) quantitatively measuring the amount of nucleic acid in the specimen;
(2) measuring the distribution of cell types existing in the specimen; and
(3) correcting the measured value of the amount of nucleic acid based on the measured value of cell distribution, wherein the specimen comprises control cells and target cells and wherein the control cell is a normal cell and the target cell is an abnormal cell; the ratio of control cells to target cells in the specimen is assayed in step (2); and the amount of nucleic acid in the target cell is determined in step (3) by correcting the measured value of the nucleic acid in the specimen using the cell ratio obtained in step (2) and the amount of nucleic acid in the control cell as previously obtained.

Preferably, the nucleic acid is mRNA.

Preferably, the method for measuring the nucleic acid in the specimen is the DNA array technique or RT-PCR.

Preferably, in step (1), mRNA isolated from the specimen or a nucleic acid product synthesized therefrom is spotted on a chip having a probe nucleic acid immobilized thereon, and the signal intensity of hybridization is detected to quantitatively measure the amount of nucleic acid in the specimen.

Preferably, in step (2), the specimen is stained, cell nuclei are extracted by graphics extraction, the image characteristics of the extracted graphics is computed, and the cell type is determined based on the image characteristics so as to assay the ratio of control cells to target cells.

Preferably, the image characteristics are at least an area, a boundary length, and a complexity value wherein;

$$\text{complexity} = \text{area}/(\text{boundary length} \times \text{boundary length}).$$

Preferably, the specimen comprises control cells and target cells, and the amount of nucleic acid in the target cell is determined in step (3) by correcting the measured value of the amount of nucleic acid in the specimen based on the following formula:

$$[\text{amount of nucleic acid in specimen} - (\text{amount of nucleic acid in control cell}) \times x]/y$$

wherein x represents the ratio of control cells in the specimen and y represents the ratio of target cells in the specimen.

Preferably, the specimen comprises a first control cell, a second control cell and target cells, and the amount of nucleic acid in the target cell is determined in step (3) by correcting the measured value of the amount of nucleic acid in the specimen based on the following formula:

$$[\text{amount of nucleic acid in specimen} - (\text{amount of nucleic acid in first control cell}) \times x - (\text{amount of nucleic acid in second control cell}) \times y]/z$$

wherein x represents a ratio of the first control cell in the specimen, y represents a ratio of the second control cell in the specimen, and z represents a ratio of the target cell in the specimen.

Detailed Description of the Invention

The embodiments of the present invention will be described below in detail.

The present invention relates to a method for quantifying the amount of nucleic acid existing in a specimen and, more specifically, the method comprises steps of:

(1) quantitatively measuring the amount of nucleic acid in the specimen;
(2) measuring the distribution of cell types existing in the specimen; and

(3) correcting the measured value of the amount of nucleic acid based on the measured value of cell distribution, wherein the specimen comprises control cells and target cells and wherein the control cell is a normal cell and the target cell is an abnormal cell; the ratio of control cells to target cells in the specimen is assayed in step (2); and the amount of nucleic acid in the target cell is determined in step (3) by correcting the measured value of the nucleic acid in the specimen using the cell ratio obtained in step (2) and the amount of nucleic acid in the control cell as previously obtained.

The method according to the present invention can be applied as a method for diagnosing disease states by measuring the amount of mRNA being expressed in the specimen.

The "control cell" used herein is preferably a cell which can be isolated as a single cell, and more particularly is a cell in which the gene expression is normal.

The "target cell" used herein is a cell in which the amount of nucleic acid (e.g., the level of gene expression) is measured. The target cell is preferably a cell which can be isolated as a single cell, and more particularly is a cell which shows abnormal gene expression (i.e., abnormal cell). Examples thereof include cancer cells.

The control cell and the target cell may be collected from the same individual or different individuals. Preferably, they are collected from the same individual.

The "specimen" used herein includes samples containing any cells having a cytoplasm and a nucleus, and examples thereof include a sample derived from a slice of tissue (tissue slice) and a sample derived from body fluids such as a peripheral blood sample and a urine sample. These cells may contain morphologically changed cells, pathological cells and the like. In the present invention, it is preferred to use a tissue slice as a specimen.

A specimen comprising control cells and target cells is preferably a "clinical sample" obtained from a patient. The clinical sample provides a rich information source regarding various states of gene network or gene expression. According to the present invention, the level of gene expression in the target cell can be analyzed. Representative examples of clinical samples include, but are not limited to, phlegm, blood, blood cell (e.g., leukocyte), tissue, or fine needle biopsy sample, urine, peritoneal effusion, pleural effusion, or cells thereof. Clinical samples may be a tissue slice such as frozen slice or formalin-immobilized slice collected for histological purpose. Another source of biological sample is cultured cells, the gene expression state of which can be controlled for the purpose of studying the relationship among genes.

The term "quantitatively measuring the amount of nucleic acid in the specimen" used herein is understood in its widest sense, and includes all the analyses including detection or quantification of nucleic acids existing in the specimen. According to an embodiment of the present invention, a sample-derived nucleic acid mixture is hybridized with a previously immobilized nucleic acid (i.e., a probe nucleic acid) to detect the presence or absence of the target nucleic acid in the sample or to measure and compare the abundance of the target nucleic acid. By utilizing the method according to the present invention, for example, the amount of mRNA expressed in a given cell or tissue can be measured.

More specifically, mRNA isolated from the sample or a nucleic acid product synthesized therefrom is spotted on a chip having a probe nucleic acid immobilized thereon, and then the signal intensity of hybridization is detected. Thus, the level of gene expression in each sample can be measured.

According to a preferred embodiment of the present invention, the nucleic acid probe is immobilized on a solid-phase support. According to a more preferred embodiment, the nucleic acid probe is immobilized on a solid-phase support as an array.

The Solid-phase supports which can be used in the present invention include any solid-phase supports such as fine particles, beads, membranes, slides, plates, and micromachined chips. The solid-phase support may be of glass, plastic, silicon, alkanethioate-derivatized gold, cellulose, low crosslinked and high crosslinked polystyrenes, silica gel, or polyamide.

A high-density array is particularly useful for monitoring the expression control in transcription, RNA processing and decomposition levels. Production and application of the high-density array for monitoring the gene expression are disclosed in, for example, International Publications Nos. WO97/10365 and WO92/10588.

Each probe nucleic acid occupies a known position on a substrate. The target nucleic acid sample (for example, mRNA prepared from a sample containing cells) is hybridized to the high-density array of the probe nucleic acid, and the amount of the target nucleic acid which was hybridized with each probe nucleic acid on the array is quantified. The method for the quantification is not particularly limited. A confocal microscope and fluorescent labeling can be used.

The high-density array is suitable for quantifying small variation in the expression level of a given gene in the presence of a large amount of heterogeneous nucleic acids. Such high-density array can be newly synthesized on a substrate or can be produced by spotting a probe nucleic acid at a specific position on a substrate.

In general, a probe nucleic acid means a deoxyribonucleotide or ribonucleotide polymer, and includes an analog of a naturally-occurring nucleotide which can function in the same manner as with a naturally-occurring nucleotide. An oligonucleotide can be used as a probe nucleic acid. The length of the oligonucleotide nucleic acid probe is not particularly limited and is generally 2 bp to several kbp or longer and preferably about 2 bp to 1000 bp. A nucleic acid probe can be cloned or synthesized by using any known technique in the art. In order to improve hybridization, a modified nucleotide analog, a related nucleotide, a peptidic nucleic acid or the like, which are absent in the natural world, can be used as a nucleic acid probe.

A probe nucleic acid can be purified from biomaterials such as bacterial plasmid containing a cloned fragment of a specific sequence. Alternatively, a probe nucleic acid can be produced by amplification of a template, and examples of suitable methods for amplifying the nucleic acid include the polymerase chain reaction and/or the *in vitro* transcription reaction.

It is also preferred to use a synthesized oligonucleotide array. The oligonucleotide array has many advantages such as high production efficiency, low variation within an array and between arrays, large information content, and high signal/noise ratio.

A particularly preferred high-density array contains different oligonucleotide probes in amounts of about 100 or more, preferably about 1,000 or more, more preferably about 16,000 or more, most preferably 65,000 or 250,000 or more, or about 1,000,000 or more, per surface area of 1 $cm^2$ or smaller. The length of the oligonucleotide probe is preferably about 5 to about 50 or about 500 nucleotides, more preferably about 10 to about 40 nucleotides, and most preferably about 15 to 40 nucleotides.

In general, the process for monitoring the gene expression comprises the steps: (a) preparing a pool of the target nucleic acids comprising an RNA transcript of at least one target gene or a nucleic acid derived from the RNA transcript; (b) hybridizing a nucleic acid sample with a high-density array of probe nucleic acid; and (c) detecting the hybridized nucleic acid and calculating a relative and/or absolute expression level. Each of these steps is described below.

<u>(a) Step of preparing a pool of target nucleic acids comprising an RNA transcript of at least one target gene or nucleic acid derived from the RNA transcript</u>

mRNA isolated from a sample and nucleic acid product synthesized therefrom includes mRNA, cDNA, and cRNA. Specific examples thereof include, but are not limited to, a gene transcript, cDNA obtained by reverse transcription of the transcript, cRNA obtained by transcription of the cDNA, DNA obtained by amplification of the gene, and RNA obtained by transcription of the amplified DNA.

A method for isolating mRNA from a sample is well-known to an ordinarily skilled person in the art. A method for isolating and purifying a nucleic acid is described in detail in, for example, "Laboratory Techniques in Biochemistry and Molecular Biology: Hybridization with Nucleic Acid Probes, Part I. Theory and Nucleic Acid Preparation" (Chapter 3, edited by P. Tijssen, Elsevier, New York (1993)).

As an example, a method for isolating mRNA from a sample is carried out by isolating total RNA from a given sample by acid guanidinium-phenol-chloroform (AGPC) extraction and isolating poly $A^+$ mRNA using oligo (dT) column chromatography or (dT) on magnetic beads (for example, see "Molecular Cloning: A Laboratory Manual, Second Edition," vols. 1 to 3, Sambrook et al., Cold Spring Harbor Laboratory (1989) or "Current Protocols in Molecular Biology," edited by Ausubel et al., Greene Publishing and Wiley-Interscience, New York (1987)).

In some cases, it is preferred to amplify the nucleic acid sample prior to hybridization. More specifically, when a quantitative result is desired, a relative frequency of nucleic acid to be amplified should be maintained or controlled in order to achieve a quantitative amplification.

The "quantitative" amplification method is well-known to an ordinarily skilled person in the art. Examples thereof include, but are not limited to, quantitative PCR, ligase chain reaction (LCR) (see Wu and Wallace, Genomics, 4: 560 (1989), Landergren et al., Science, 241: 1077 (1988), and Barringer et al., Gene 89: 117 (1990)), transcription amplification (see Kwoh et al., Proc. Natl. Acad. Sci. USA 86: 1173 (1989)), and self-sustaining sequence replication (Guatelli et al., Proc. Natl. Acad. Sci. USA 87: 1864 (1990)).

<u>(b) Step of hybridizing a nucleic acid sample with a high-density array of probe nucleic acid</u>

A high-density array generally contains several probe nucleic acids to be specifically hybridized with a sequence of interest. Further, an array may contain one or more control probes.

A probe nucleic acid is an oligonucleotide having a length of about 5 to about 45 or about 5 to about 500 nucleotides, more preferably about 10 to about 40 nucleotides, and most preferably about 15 to about 40 nucleotides. According to another particularly preferred embodiment, the length of the probe is 20 to 25 nucleotides. In another preferred embodiment, the probe nucleic acid is either double-strand or single-strand DNA. The DNA can be isolated or cloned from naturally-occurring sources or can be amplified from naturally-occurring sources using a native nucleic acid as a template.

In addition to a probe nucleic acid to be bound to the target nucleic acid of interest, a high-density array can contain several control probes (for example, a control for standardization, a control for expression level, or a control for mismatch). A control for standardization is a probe nucleic acid which is complementary with the labeled control nucleic acid to be added to a nucleic acid sample. After hybridization, a signal obtained from the control for standardization can be used as a control regarding the variation of hybridization conditions, labeling intensity, "reading" efficiency and other factors which may vary the signal of complete hybridization depending on each array. For example, signals read from all the other probes of the array (e.g., fluorescence intensity) are divided by a signal of the control probe (e.g., fluorescence intensity), thereby normalizing the measured value.

A control for expression level is a probe which specifically hybridizes with genes constitutively expressed in a biological sample. Practically, any gene which is constitutively expressed provides a suitable target of the control for expression level. Typically, a control probe for expression level has a sequence which is complementary with a subsequence of constitutively expressed "house-keeping genes" which include, but are not limited to, β-actin gene, transferin receptor gene, and GAPDH gene.

A control for mismatch may be provided in addition to a probe nucleic acid, a control for expression level or a control for standardization of the target gene. The control for mismatch is a probe nucleic acid which is the same as a corresponding test or control probe with the exception that one or more mismatch bases are present. A mismatching base is selected so as not to be complementary with a corresponding base in the target sequence with which the probe is specifically hybridized. One or more mismatches can be selected in such a way that a probe nucleic acid or a control probe is expected to hybridize with the target sequence but a mismatch probe is expected not to hybridize therewith (or to hybridize at a considerably low level) under adequate hybridization conditions (e.g., a stringent condition).

Accordingly, the mismatch probe provides a control for non-specific binding or cross-hybridization to a nucleic acid other than the corresponding target in the sample. More specifically, the mismatch probe indicates whether hybridization is specific or not.

(c) Step of detecting the hybridized nucleic acid and calculating a relative and/or absolute expression level

Nucleic acid hybridization comprises a step of contacting a probe nucleic acid with a target nucleic acid under such a condition that the probe nucleic acid and the target sequence complementary therewith can form a stable hybrid through complementary base pairing. Thereafter, nucleotide acids which do not form hybrid are washed out to leave the hybridized nucleic acid. This nucleic acid can be detected generally by detecting a conjugated detectable label. An ordinarily skilled person in the art can adequately select a hybridization condition. A method for optimizing a hybridization condition is well-known in the art (for example, see "Laboratory Techniques in Biochemistry and Molecular Biology," vol. 24, "Hybridization with Nucleic Acid Probes," edited by P. Tijssen, Elsevier, New York (1993)).

According to a preferred embodiment, hybridized nucleic acids are detected by detecting one or more labels conjugated to a sample-derived nucleic acid. Labeling can be carried out in accordance with any well-known method in the art. In a preferred embodiment, a label is incorporated simultaneously at the amplification step at the time of nucleic acid sample preparation. For example, a labeled amplification product can be obtained by polymerase chain reaction (PCR) using a labeled primer or a labeled nucleotide.

A label can also be directly added to an original nucleic acid sample (e.g., mRNA, poly A mRNA, cDNA and the like) or to an amplification product after the completion of amplification. Methods for binding a label to a nucleic acid are well-known in the art, and examples thereof include nick translation and end labeling (e.g., labeled RNA was used) in which a nucleic acid is phosphorylated and a nucleic acid linker is then conjugated (ligated), thereby binding a sample nucleic acid to a label (e.g., fluorescent material).

Detectable labels which can be preferably used in the present invention include any labeling substances which are detectable by a spectroscopic, photochemical, biochemical, immunological, electrical, optical, or chemical technique. Examples thereof include biotin for staining with a labeled streptoavidin conjugate, magnetic beads (e.g., Dynabeads (registered trademark)), fluorescent dyes (e.g., Cy5, Cy3 (Amersham Biotech), fluorescein, Texas Red, Rhodamine, green fluorescent protein (GFP)), radioactive labels (e.g., $^{3}H$, $^{125}I$, $^{35}S$, $^{14}C$, or $^{32}P$), enzymes (e.g., horse radish peroxidase, alkaline phosphatase, and other enzymes commonly used in ELISA), colorimetric labels such as colloidal gold and colored glass, and plastic beads (e.g., polystyrene, polypropylene, and latex).

Methods for detecting such labels are well-known to an ordinarily skilled person in the art. For example, a radioactive label can be detected by using a photographic film or a scintillation counter, and a fluorescent label can be detected by detecting light emissions using a photodetector. An enzyme label is generally detected by providing a substrate to an enzyme and then detecting reaction products generated by the action of the enzyme on the substrate, and a colorimetric label can be detected by visually detecting the colored label.

Preferably, fluorescent label can be easily added during an *in vitro* transcription reaction. According to a preferred embodiment, Cy5-labeled UTP and CTP are incorporated into RNA generated during the *in vitro* transcription reaction as described above.

Methods for detecting a labeled target (sample) nucleic acid which has been hybridized with a probe in a high-density array, are well-known in the art.

According to a preferred embodiment, a target nucleic acid is fluorescently labeled and the location of the label on the probe array is detected by a fluorescence microscope. The hybridized target nucleic acid is excited with a light source having an excitation wavelength of the specific fluorescent label, and the fluorescence of the resultant emission wavelength is detected. According to a particularly preferred embodiment, the excitation light source is a laser suitable for exciting the fluorescent label.

A confocal microscope is automated together with a computer control stage, and the entire high-density array can be automatically scanned. Similarly, a phototransducer connected to an automatic data collecting system (e.g., a photomultiplier, a solid array, and a CCD camera) is provided on a microscope, so that fluorescent signals generated by hybridizaton with each oligonucleotide probe on the array can be automatically recorded. Such an automated system is described in, for example, USP No. 5,143,854 and WO 92/10092.

In general, the methods for evaluating the results of hybridization are different depending on the properties of specific probe nucleic acids used and the provided control. According to the simplest embodiment, the fluorescent intensity of each probe is simply quantified. This can be accomplished by merely measuring the intensity of probe signals at each position (indicating different probes) on the high-density array (e.g., when the label is a fluorescent label, the level (intensity) of fluorescence generated by excitation irradiation at each immobilized position on the array is detected). By comparing the absolute intensity of the array which was hybridized with the nucleic acid of a "test" sample with the intensity generated by the "control" sample, the relative expression of the nucleic acid which has been hybridized with each probe can be measured.

In the present invention, distribution of cell types existing in a specimen is measured. More specifically, by measuring the ratio of control cells to target cells in a specimen comprising control cells and target cells, the distribution of cell types can be measured. Methods for measuring the cell ratio are not particularly limited and any desired method can be employed. Distribution of cell types in the specimen (e.g., a cell slice) can be assayed by visual observation by a human. Alternatively, it can be automatically assayed using instruments.

For example, cells are stained, and morphological information is obtained from the different stained patterns. Then, based on this information, the cell type is identified and the number of cells for each type is counted, so as to determine the cell ratio.

Systems for simultaneously analyzing cell distributions and their images at high speed and high capacity include a flow imaging cytometer system (FIC) (Cytometry, 21: p. 129-132 (1995)).

In the present invention, a sample containing several types of cells may be stained with a stain solution which is excitable at a specific wavelength to provide a contrast between nuclei and cytoplasms. Subsequently, the stained cell material is introduced into a flow imaging cytometer equipped with a light source to obtain cell distribution information and cell image information.

A method for staining a cell-containing sample with a stain solution can be carried out by, for example, adding a solution containing a suitable dye into the cell-containing sample and stirring the resultant mixture, thereby staining the nucleus and the cytoplasm at high contrast. In this case, staining temperature and staining period are not particularly limited, and may be about 15 to 40°C and about 1 to 60 minutes respectively.

In the present invention, double staining may be carried out by different staining methods besides the above-described staining methods. Examples of different staining methods include staining which employs a stain solution containing at least one dye capable of staining DNA, and staining which utilizes a labeled antibody or a fluorescent dye-labeled material. The dyes capable of staining DNA include propidium iodide (PI), ethidium bromide (EB), ethidium-acridine heterodimer, ethidium diazide, and ethidium monoazide.

Specific examples of methods for measuring the cell ratio is described below in detail.

First, a digital image of an HE-stained (hematoxylin/eosin-stained) tissue slice is obtained by a CCD camera which has been connected to the eyepiece portion of a microscope. The obtained image was subjected to the following processing to count the number of cells for each cell type.

(1) Preparation of image showing intensity distribution

Since the nuclei were stained blue with HE-staining, the image was converted to show the intensity distribution of blue in a color image based on the following conversion formula.

<Conversion formula>

Components of red, green, and blue at the coordinate (x, y) in the original image are respectively set to be r (x, y), g (x, Y), and b (x, y), and the pixel value of the coordinate (x, y) of the image showing the intensity distribution are set to be P (x, y):

$$P(x,y) = \alpha \times \frac{b(x,y)}{r(x,y) + g(x,y) + b(x,y)}$$

wherein $\alpha$ is a constant for normalization.

(2) Graphic extraction of cell nuclei from image

In order to extract the portions which contain only cell nuclei, the image showing the intensity distribution of blue is two-valued with an appropriate threshold. Graphics extraction which is referred to as "labeling" is applied on the two-valued image to extract all of the connected components constituted by one or more same pixels connected with each other.

(3) Calculation of image characteristics, and removal of trash

Image characteristics of the extracted graphics are calculated. For the image characteristics, values of an area, a boundary length and a complexity value can be calculated. The complexity value is defined by the following equation. The rounder the shape is, the larger the value is.

$$\text{Complexity} = \text{area} / (\text{boundary length} \times \text{boundary length}).$$

(4) Removal of noise

Graphics having an area smaller than a given level or a complexity lower than a given level is defined as a noise component and removed from a list.

(5) Determination of cell type

The information of the shapes and areas of the remaining graphics is applied to a predefined standard to determine the types of cell nucleus in the image.

(6) Determination of cell ratio

The number of cells is counted for each cell type determined in (5) above, thereby obtaining the cell ratio.

In the present invention, the measured value of the amount of nucleic acid in a specimen comprising control cells and target cells is corrected by using the cell ratio obtained in step (2) above, thereby determining the amount of nucleic acid in the target cell.

Feasible methods for data correction include a method in which data of step (1) is indicated together with data of step (2), as well as a method in which data of step (2) is mathematically corrected using data of step (1).

Data on the amount of nucleic acid can be corrected by using a suitable formula. Preferably, but not particularly limited to, the amount of nucleic acid in the specimen is corrected based on the following formula, thereby determining the amount of nucleic acid in the target cell:

$$[\text{amount of nucleic acid in specimen} - (\text{amount of nucleic acid in control cell}) \times x]/y$$

wherein x represents the ratio of control cells in the specimen and y represents the ratio of target cells in the specimen.

The data on the amount of nucleic acid in the control cell can be used as needed if the data is previously obtained and stored.

In the present invention, the control cell may be one type or several types. For example, when two types of control samples are present, the specimen comprises a first control cell, a second control cell, and target cells. In such a case, in step (3), the measured value of the amount of nucleic acid in the specimen is corrected based on the following formula to determine the amount of nucleic acid in the target cell:

$$[\text{amount of nucleic acid in specimen} - (\text{amount of nucleic acid in first control cell}) \times x - (\text{amount of nucleic acid in second control cell}) \times y]/z$$

wherein x represents a ratio of the first control cell in the specimen, y represents a ratio of the second control cell in the specimen, and z represents a ratio of the target cell in the specimen.

The present invention is described in more detail with reference to the following examples, although the present invention is not limited by these examples.

Examples

[Example 1: Correction of mRNA expression data by the assay of cell distribution]

(1): Preparation of enzyme-labeled cDNA

(A) Preparation of single-strand cDNA

Human liver-derived cell lines (HEPG2) and/or human small intestine-derived cell lines (Caco-2) were mixed as shown in the table below in such a way that the total number of cells is 100,000.

Table 1

| | Number of HEPG2 cells | Number of Caco-2 cells | Number of total cells |
|---|---|---|---|
| Sample 1 | 100,000 | 0 | 100,000 |
| Sample 2 | 0 | 100,000 | 100,000 |
| Sample 3 | 40,000 | 60,000 | 100,000 |

Total RNAs of Samples 1, 2 and 3 were mixed with 500 ng of gene-specific primer (as below) mixture, and RNAse free sterilized water was added to bring the mixture to 12 μl. The mixture was heated at 70°C for 10 minutes and then quenched in ice bath. The content was collected at the bottom of the tube by a centrifuge, then 4 μl of 5x first strand synthesizing buffer (250 mM Tris-HCl (pH 8.3), 375 mM KCl, and 15 mM $MgCl_2$), 2 μl of 0.1 M DTT, 1 μl of mixture of 10 mM dATP, dCTP and dGTP, 5 μl of 1 mM dUTP, and 5 μl of 1 mM Fluorescein-dUTP(Amersham) were added and mixed, and the mixture was incubated at 42°C for 2 minutes. Subsequently, 200 units of reverse transcriptase SuperScriptII (Gibco BRL) were added and mixed, and the resultant mixture was incubated at 42°C for 30 minutes, followed by heating at 70°C for 15 minutes. Finally, 2 units of *Escherichia coli*-derived RNAse H were added and mixed, and the mixture was then incubated at 37°C for 20 minutes.

Primers which were used (31 types, SEQ ID NOS: 1 to 31 of the Sequence Listing):

HSA1L: AAAGGAGTTC CGGGGCATAAAAG
HSA2L: TGGCAGCATT CCTTGTGG
HSA3L: TCTCAGCAGC AGCACGAC
HSA4L: AACATTTGCT GCCCACTTTT CCTA
HSA5L: CTCGGCAAAG CAGGTCT
HSA6L: TTAATTAGCC CACAGAAACT
AA2L: GCAAAGAGTG GGTAGGGACA GGAG
GP1L: CAGGGGGCAG TCTTGGCACA CC
GP2L: GAAGGTGTGG CGCAGGTCGT AGTG
GP3L: TCAGGCATT TCATTAACAG GCACA T
REP1L: CCTCCATCGG ACATGTCACA ATAAAC
REP2L: AAGTAACAAA GGCAAGTGAG AAGAATG
REP3L: ACCCATGCCA GGTGTACCAG ACAATC
REP4L: TTGGGGAATA TTCAACTCGT AGAAAT
ECD1L: GTGCTGGGTG AACCTTCTGA TGCTAA

ECD2L: CCAGCCTGGC CGATAGAATG AGA
ECD3L: ACTTGAGCCC AGGAGTTTGA GACCA
ECD4L: GGGGGCCAAG GCAGAAGGAT T
ECD5L: TGGATAGCTG CCCATTGCAAGTTACA
ECD6L: GGGCCACATT TTCTTCTTGC TCCTA
ECD7L: TCCACCCCCAAAGAAAATAC
NET1L: TCTTTCTTGA TGGCAGGCAC TACC
NET2L: CAGTTCCCAT GTGGCAGCAG TAGTG
NET3L: CAGAGGCTCT GCTGATTTCA CTTATG
EF1L: CACCAACACC AGCAGCAACA ATC
EF2L: AGGGCTTGTC AGTTGGACGA GTT
EF3L: GGCGATCAAT CTTTTCCTTC AGC
EF4L: TGAGACCGTT CTTCCACCAC TGATTA
ACT1L: CGCGGTTGGC CTTGGGGTTC AG
ACT2L: GCCTAGAAGC ATTTGCGGTG GACGAT
ACT3L: GGCTGCCTCC ACCCACTCC

(2) Hybridization

Serum albumin, α-2-HS glycoprotein, HFREP-1, E-cadherin, tetraspan NET-1, β-actin, and an EF1-α gene fragment (about 500 bp) were prepared by PCR, purified and mixed. The mixture was heated to 95°C and quenched. Subsequently, 1 μl of the product (corresponding to 2 ng DNA) was spotted on a nylon membrane HyBond N+, air dried, and irradiated with UV. The DNA-spotted membrane was immersed in a hybridization buffer (0.5 M Church-phosphate buffer (pH 7.2), 1mM EDTA, 7% SDS) and shaken at 60°C for 30 minutes. Thereafter, fluorescein-labeled probes prepared in Example 1 were added to bring the final concentration to 5 ng/ml, and the mixture was further shaken at 60°C for 16 hours.

(3) Detection

The above-described membrane was immersed in washing solution (1% SSC, 0.1 % SDS) and shaken at 60°C for 10 minutes. This procedure was repeated three times. The membrane was rinsed once with buffer A (100 mM Tris-HCl (pH 7.5), 600 mM NaCl) and immersed in a solution which was prepared by diluting Liquid Block (Amersham) by 20-fold with the above buffer A, for 30 minutes. The membrane was then immersed in a solution which was prepared by diluting HRP-labeled anti-fluoresceine antibody by 1000-fold with buffer A containing 0.5% BSA, for 30 minutes, and then in 0.1% Tween 20 for 10 minutes × 3 times. This membrane was placed on a wrap film, and a sufficient amount of ECL detection reagent attached to the kit was added dropwise. 3 minutes later, the membrane was covered with a wrap, and light emission was detected and recorded using LAS1000.

The emission intensity for each gene is shown in the table below. Emission intensity is indicated by 5 grades (larger number indicates stronger emission intensity).

Table 2

| Gene | Sample 1 | Sample 2 | Sample 3 | Corrected value for sample 3 |
|---|---|---|---|---|
| Serum albumin | 5 | 1 | 3 | 2 |
| α-2-HSGP | 4 | 1 | 3 | 2 |
| HFREP | 2 | 1 | 1 | 0 |
| E-cadherin | 1 | 3 | 2 | 3 |
| Tetraspan NET-1 | 1 | 3 | 3 | 4 |
| β-actin | 3 | 2 | 3 | 3 |
| EF1-α | 5 | 4 | 5 | 5 |

In the table, the corrected value for sample 3 is calculated by the following formula:

$$(\text{data of Sample 3} - \text{data of Sample 1} \times 0.4) \div 0.6$$

The measured value of sample 3 before correction (a mixture of human small intestine-derived cell lines and human liver-derived cell lines (4 : 6)) does not accurately indicate the mRNA expression pattern of human small intestine-derived cell lines. However, by correcting the value on as described above, it is clear that more accurate analysis of mRNA expression can be carried out.

This result demonstrates that correction of data on mRNA expression based on the ratio of existing cells leads to more accurate analysis of mRNA expression.

[Example 2: Cell counting of tissue slice]

A digital image of HE-stained tissue slice of a skin surface layer was obtained by a CCD camera which has been connected to the eyepiece portion of a microscope. The obtained image was subjected to the following processing to count the number of cells for each cell type.

(1) Preparation of image showing intensity distribution

Since the nuclei were stained blue with HE-staining, the image was converted to show the intensity distribution of blue in a color image based on the following conversion formula.

<Conversion formula >

Components of red, green, and blue at the coordinate (x, y) in the original image are respectively set to be r (x, y), g (x, Y), and b (x, y), and the pixel value of the coordinate (x, y) of the image showing the intensity distribution are set to be P (x, y):

$$P(x, y) = \alpha \times \frac{b(x, y)}{r(x, y) + g(x, y) + b(x, y)}$$

wherein $\alpha$ is a constant for normalization.

(2) Graphic extraction of cell nuclei from image

In order to extract the portions which contain only cell nuclei, the image showing the intensity distribution of blue is two-valued with an appropriate threshold. Graphics extraction which is referred to as "labeling" is applied on the two-valued image to extract all of the connected components constituted by one or more same pixels connected with each other.

(3) Calculation of image characteristics, and removal of trash

Image characteristics of the extracted graphics are calculated. In this case, values of an area, a boundary length and a complexity value were calculated. The complexity value is defined by the following equation. The rounder the shape is, the larger the value is.

<Definition of complexity>

$$\text{Complexity} = \text{area} / (\text{boundary length} \times \text{boundary length}).$$

(4) Removal of noise

Graphics having an area smaller than a given level or a complexity lower than a given level is defined as a noise component and removed from a list.

(5) Determination of cell type

Information of the shapes and areas of the remaining graphics is applied to the following table to determine the type of cell nucleus. The shape was judged by the complexity.

Table 3

| Shape | Area | Deduced cell type |
|---|---|---|
| Round | Large | Normal cell |
| Round | Small | Lymphocyte |
| Elongate | - | Fibroblast |

Experimental Results

The above processing was carried out for an image of tissue slices which were used as sample. As a result, the cell distribution in this tissue slice was found to include normal cells (65%), lymphocytes (32%), and fibroblasts (3%).

Effect of the Invention

The present invention provides a method for measuring the expression level of genes in the target cell existing in a sample containing several types of cells. More particularly, the present invention provides a method for measuring the expression level of genes in the target cell without isolating target cells from control cells in the sample comprising one or more types of control cells and one type of target cell.

## Claims

1. A method for quantifying the amount of nucleic acid existing in a specimen which comprises steps of:

(1) quantitatively measuring the amount of nucleic acid in the specimen;
(2) measuring the distribution of cell types existing in the specimen; and
(3) correcting the measured value of the amount of nucleic acid based on the measured value of cell distribution, wherein the specimen comprises control cells and target cells and wherein the control cell is a normal cell and the target cell is an abnormal cell; the ratio of control cells to target cells in the specimen is assayed in step (2); and the amount of nucleic acid in the target cell is determined in step (3) by correcting the measured value of the nucleic acid in the specimen using the cell ratio obtained in step (2) and the amount of nucleic acid in the control cell as previously obtained.

2. The method according to claim 1 wherein the nucleic acid is mRNA.

3. The method according to claim 1 wherein the method for measuring the nucleic acid in the specimen is the DNA array technique or RT-PCR.

4. The method according to claim 1 wherein, in step (1), mRNA isolated from the specimen or a nucleic acid product synthesized therefrom is spotted on a chip having a probe nucleic acid immobilized thereon, and the signal intensity of hybridization is detected to quantitatively measure the amount of nucleic acid in the specimen.

5. The method according to claim 1 wherein, in step (2), the specimen is stained, cell nuclei are extracted by graphics extraction, the image characteristics of the extracted graphics is computed, and the cell type is determined based on the image characteristics so as to assay the ratio of control cells to target cells.

6. The method according to claim 5 wherein the image characteristics are at least an area, a boundary length, and a complexity value wherein;

$$\text{complexity} = \text{area}/(\text{boundary length} \times \text{boundary length}).$$

7. The method according to claim 1 wherein the specimen comprises control cells and target cells, and the amount of nucleic acid in the target cell is determined in step (3) by correcting the measured value of the amount of nucleic acid in the specimen based on the following formula:

$$[\text{amount of nucleic acid in specimen} - (\text{amount of nucleic acid in control cell}) \times x]/y$$

wherein x represents the ratio of control cells in the specimen and y represents the ratio of target cells in the specimen.

8. The method according to claim 1 wherein the specimen comprises a first control cell, a second control cell and target cells, and the amount of nucleic acid in the target cell is determined in step (3) by correcting the measured value of the amount of nucleic acid in the specimen based on the following formula:

$$[\text{amount of nucleic acid in specimen} - (\text{amount of nucleic acid in first control cell}) \times x - (\text{amount of nucleic acid in second control cell}) \times y]/z$$

wherein x represents a ratio of the first control cell in the specimen, y represents a ratio of the second control cell in the specimen, and z represents a ratio of the target cell in the specimen.

## Patentansprüche

1. Verfahren zum Quantifizieren der Menge an Nukleinsäure, die in einer Probe vorliegt, umfassend die Stufen:

   (1) quantitatives Messen der Menge an Nukleinsäure in der Probe;
   (2) Messen der Verteilung von Zelltypen, die in der Probe vorliegt; und
   (3) Korrigieren des gemessenen Werts der Menge an Nukleinsäure basierend auf dem gemessenen Wert der Zellverteilung, wobei die Probe Kontrollzellen und Zielzellen umfasst und wobei die Kontrollzelle eine normale Zelle ist und die Zielzelle eine abnormale Zelle ist; das Verhältnis von Kontrollzellen zu Zielzellen in der Probe in Stufe (2) untersucht wird; und die Menge an Nukleinsäure in der Zielzelle in Stufe (3) bestimmt wird durch Korrigieren des gemessenen Werts der Nukleinsäure in der Probe unter Verwendung des Zellverhältnisses, das in Stufe (2) erhalten wird, und der Menge an Nukleinsäure in der Kontrollzelle, wie zuvor erhalten.

2. Verfahren nach Anspruch 1, wobei die Nukleinsäure mRNA ist.

3. Verfahren nach Anspruch 1, wobei das Verfahren zum Messen der Nukleinsäure in der Probe die DNA-Array-Technik oder RT-PCR ist.

4. Verfahren nach Anspruch 1, wobei in Stufe (1) mRNA, isoliert aus der Probe, oder ein Nukleinsäureprodukt, das davon ausgehend synthetisiert wird, auf einem Chip mit einer darauf immobilisierten Sondennukleinsäure platziert wird und die Signalintensität der Hybridisierung detektiert wird, um die Menge an Nukleinsäure in der Probe quantitativ zu messen.

5. Verfahren nach Anspruch 1, wobei in Stufe (2) die Probe gefärbt wird, Zellkerne mittels Graphikverarbeitung extrahiert werden ("extracted by graphics extraction"), die Bildeigenschaften der verarbeiteten Graphiken berechnet werden und der Zelltyp basierend auf den Bildeigenschaften bestimmt wird, um so das Verhältnis von Kontrollzellen zu Zielzellen zu untersuchen.

6. Verfahren nach Anspruch 5, wobei die Bildeigenschaften wenigstens eine Fläche, eine Randlänge ("boundary length") und ein Komplexitätswert sind, wobei:

$$\text{Komplexität} = \text{Fläche}/(\text{Randlänge} \times \text{Randlänge}).$$

7. Verfahren nach Anspruch 1, wobei die Probe Kontrollzellen und Zielzellen umfasst und die Menge an Nukleinsäure in der Zielzelle in Stufe (3) bestimmt wird durch Korrigieren des gemessenen Werts der Menge an Nukleinsäure in der Probe, basierend auf der folgenden Formel:

$$[\text{Menge an Nukleinsäure in der Probe} - (\text{Menge an Nukleinsäure in Kontrollzelle}) \times x]/y$$

   wobei x den Anteil von Kontrollzellen in der Probe darstellt und y den Anteil von Zielzellen in der Probe darstellt.

8. Verfahren nach Anspruch 1, wobei die Probe eine erste Kontrollzelle, eine zweite Kontrollzelle und Zielzellen umfasst und die Menge an Nukleinsäure in der Zielzelle in Stufe (3) bestimmt wird durch Korrigieren des gemessenen Werts der Menge an Nukleinsäure in der Probe, basierend auf der folgenden Formel:

$$[\text{Menge an Nukleinsäure in der Probe} - (\text{Menge an Nukleinsäure in der ersten Kontrollzelle}) \times x - (\text{Menge an Nukleinsäure in der zweiten Kontrollzelle}) \times y]/z$$

wobei x einen Anteil der ersten Kontrollzelle in der Probe darstellt, y einen Anteil der zweiten Kontrollzelle in der Probe darstellt und z einen Anteil der Zielzelle in der Probe darstellt.

**Revendications**

1. Un procédé pour déterminer la quantité d'acide nucléique présente dans un échantillon comprenant les étapes consistant

   (1) à mesurer quantitativement la quantité d'acide nucléique dans l' échantillon;
   (2) à mesurer la distribution des types de cellules présents dans l'échantillon et
   (3) à corriger la valeur mesurée de la quantité d'acide nucléique basée sur la valeur mesurée de la distribution des cellules,

   dans lequel l'échantillon comprend des cellules témoins et des cellules cibles et dans lequel la cellule témoin est une cellule normale et la cellule cible est une cellule anormale; le rapport cellules témoins - cellules cibles dans l'échantillon est analysé à l'étape (2) et la quantité d'acide nucléique dans la cellule cible est déterminée à l'étape (3) en corrigeant la valeur mesurée de l'acide nucléique dans l'échantillon utilisant le rapport des cellules obtenu à l'étape (2) et la quantité d'acide nucléique dans la cellule témoin obtenue auparavant.

2. Le procédé selon la revendication 1, dans lequel l'acide nucléique est l'ARNm.

3. Le procédé selon la revendication 1, dans lequel le procédé pour mesurer l'acide nucléique dans l'échantillon est la technique de l'Array d'ADN ou RT-PCR.

4. Le procédé selon la revendication 1, dans lequel à l'étape (1) ARNm isolé de l'échantillon ou un produit d'acide nucléique synthétisé de celui-ci est appliqué sur une puce d'ADN, sur laquelle un échantillon d'acide nucléique est immobilisé et l'intensité de signal est détectée pour mesurer quantitativement la quantité d'acide nucléique dans l'échantillon.

5. Le procédé selon la revendication 1, dans lequel à l'étape (2) l'échantillon est coloré, les noyaux de cellules sont extraits par extraction graphique, les caractéristiques d'image des graphiques extraits sont informatisées et le type de cellules est déterminé sur la base des caractéristiques d'image pour analyser le rapport cellules témoins - cellules cibles.

6. Le procédé selon la revendication 5, dans lequel les caractéristiques d'image sont au moins la face, la ligne de démarcation et la valeur de complexité, dans lequel

   complexité = face/(ligne de démarcation × ligne de démarcation).

7. Le procédé selon la revendication 1, dans lequel l'échantillon comprend des cellules témoins et des cellules cibles et la quantité d'acide nucléique dans la cellule cible est déterminée à l'étape (3) en corrigeant la valeur mesurée de la quantité d'acide nucléique dans l'échantillon sur la base de la formule suivante:

   [quantité d'acide nucléique dans l'échantillon - (quantité d'acide nucléique dans la cellule témoin) × x]/y

   dans laquelle x représente la proportion des cellules témoins dans l'échantillon et y représente la proportion des cellules cibles dans l'échantillon.

8. Le procédé selon la revendication 1, dans lequel l'échantillon comprend une première cellule témoin, une deuxième cellule témoin et des cellules cibles, et la quantité d'acide nucléique dans la cellule cible est déterminée à l'étape (3) en corrigeant la valeur mesurée de la quantité d'acide nucléique dans l'échantillon sur la base de la formule

suivants :

[quantité d'acide nucléique dans l'échantillon - (quantité d'acide nucléique dans la première cellule témoin) × x - (quantité d'acide nucléique dans la deuxième cellule témoin) × y]/z

dans laquelle x représente la proportion de la première cellule témoin dans l'échantillon, y représente la proportion de la deuxième cellule témoin dans l'échantillon et z représente la proportion de la cellule cible dans l'échantillon.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9710365 A
- WO 9210588 A
- US 5143854 A
- WO 9210092 A


### Non-patent literature cited in the description


- **DERIESIL, JL et al.** *Science,* 1997, vol. 278, 680-686
- **LASHKARI DA et al.** *Proc. Natl. Acad. Sci. USA,* 1997, vol. 94, 13057-13062
- **DE BLESER, P.J. et al.** *Journal of Hepatology,* 1997, vol. 26, 886-893
- Laboratory Techniques in Biochemistry and Molecular Biology: Hybridization with Nucleic Acid Probes, Part I. Theory and Nucleic Acid Preparation. Elsevier, 1993
- **SAMBROOK et al.** Molecular Cloning: A Laboratory Manual, Second Edition. Cold Spring Harbor Laboratory, 1989, vol. 1 to 3
- Current Protocols in Molecular Biology. Greene Publishing and Wiley-Interscience, 1987
- **WU ; WALLACE.** *Genomics,* 1989, vol. 4, 560
- **LANDERGREN et al.** *Science,* 1988, vol. 241, 1077
- **BARRINGER et al.** *Gene,* 1990, vol. 89, 117
- **KWOH et al.** *Proc. Natl. Acad. Sci. USA,* 1989, vol. 86, 1173
- Hybridization with Nucleic Acid Probes. Laboratory Techniques in Biochemistry and Molecular Biology. Elsevier, 1993, vol. 24
- *Cytometry,* 1995, vol. 21, 129-132